# EUROPEAN PATENT APPLICATION

(11) **EP 3 138 493 A1**
(43) Date of publication of application: **08.03.2017**
(21) Application number: 15786271.5
(22) Date of filing: 29.04.2015
(51) Int. Cl.: A61B 5/1455, A61B 5/0402, A61B 5/05, A61B 5/08

(54) **BLOOD SUGAR MEASUREMENT METHOD AND BLOOD SUGAR MEASUREMENT DEVICE USING MULTIPLE BIOLOGICAL SIGNALS**

(30) Priority: 29.04.2014 KR 20140051420
(71) Applicant: Huinno, Co., Ltd., Seongnam-si, Gyeonggi-do 463-400 (KR)
(72) Inventor: GIL, Yeong Joon, Busan 612-740 (KR)
(74) Representative: Glawe, Delfs, Moll
(86) International application number: PCT/KR2015/004324
(87) International publication number: WO 2015/167251

(57) **Abstract**

Provided is real-time blood glucose measurement using multiple body signals, and more particularly, a blood glucose measurement method and apparatus using multiple body signals, the method and apparatus employed to estimate blood glucose in a noninvasive manner by using a change in the absorbance of glucose according to a blood glucose level and correct the estimated blood glucose using different types of blood glucose measurement methods. The blood glucose measurement apparatus comprises: a main body which can be worn on a human body; a first light source unit which is provided on an inner surface of the main body and contacts the human body wearing the main body; a second light source unit which is provided on the inner surface of the main body and irradiates light in the same direction as the first light source unit; a light receiving unit which receives light from the first light source unit and the second light source unit; and a calculation unit which calculates a glucose level in blood based on the amount of light received by the light receiving unit and measures blood glucose using the calculated glucose level in the blood.

## Description

### [Technical Field]

The present invention relates to real-time blood glucose measurement using multiple body signals, and more particularly, to a blood glucose measurement method and apparatus using multiple body signals, the method and apparatus employed to estimate blood glucose in a noninvasive manner by using a change in the absorbance of glucose according to a blood glucose level and correct the estimated blood glucose using different types of blood glucose measurement methods.

### [Background Art]

Diabetes is a disease in which sugar accumulates in blood because a hormone called insulin is not secreted enough by the pancreas or fails to work properly. Diabetes causes various complications including high blood pressure, kidney failure, and damage to the eyes. Since there is no complete treatment for diabetes, it is important to maintain an appropriate glucose level in blood through dietary treatment, exercise treatment, and the injection of insulin. For the management of blood glucose levels, the accurate measurement of blood glucose is essential.

To measure blood glucose levels, there have been proposed a number of methods including a method based on the reducibility of glucose, a method using a direct reaction of sugar in an acid condition, and a method using an enzymatic reaction of glucose. In addition, methods used in clinical medicine include a method in which blood drawn from a finger or a toe is made to react with glucose oxidase, the degree of coloring is measured by utilizing a coloring reaction dependent on the concentration of glucose in blood, and the measured degree of coloring is converted into a blood glucose level. However, these invasive blood sugar measurement methods require diabetic patients to endure the pain of blood extraction, which is required a number of times in a day, and put the diabetic patients at risk of possible infection at the time of blood extraction.

Accordingly, an electrochemical method is currently the most widely used blood glucose measurement method. In this method, blood drawn is made to react with an enzyme, and then a blood glucose level is measured by measuring the amount of current flowing in response to a predetermined voltage applied to the blood. However, this method requires not only the pain of blood drawing for each measurement but also the continuous purchase of a disposable strip for blood glucose measurement.

To solve these problems, many noninvasive blood glucose measurement techniques have been developed. These techniques measure blood glucose levels by irradiating infrared light to the skin and then analyzing the degree of infrared light absorbed or scattered in the skin. However, since measured blood glucose levels vary widely according to the measurement location on the skin, the temperature of the skin, etc., it is difficult to put these techniques to practical use.

In this regard, there is a need for a noninvasive blood glucose measurement means that can accurately measure blood glucose levels even when affected by changes in environment.

### [Citation List]

### [Patent Literature]

Patent Publication No. 2003-0019927: Blood Glucose Measurement Apparatus and Method Using the Amount of Glucose Accumulated

### [Disclosure]

### [Technical Problem]

The present invention is intended to solve problems of a conventional blood pressure monitoring system. It is an objective of the present invention to provide a blood glucose measurement method and apparatus using multiple body signals, the method and apparatus capable of continuously monitoring blood glucose levels in a noninvasive manner by using a blood glucose sensor provided in a main body wearable on a human body.

It is another objective of the present invention to provide a blood glucose measurement method and apparatus using multiple body signals, the method and apparatus capable of measuring blood glucose levels by measuring the degree of light absorbed in a human body from among light irradiated from a light source provided in a main body wearable on the human body.

It is another objective of the present invention to provide a blood glucose measurement method and apparatus using multiple body signals, the method and apparatus capable of measuring blood glucose levels by detecting body signals, such as electrocardiogram (ECG), photoplethysmography (PPG), saturation of peripheral oxygen (SpO₂), galvanic skin reflex (GSR), body temperature and breathing pattern, and using the detected body signals as auxiliary information.

However, the objectives of the present invention are not restricted to the one set forth herein. The above and other objectives of the present invention will become more apparent to one of ordinary skill in the art to which the present invention pertains by referencing a detailed description of the present invention given below.

### [Technical Solution]

According to an aspect of the present invention, there is provided blood glucose measurement apparatus using multiple body signals. The apparatus includes: a main body which can be worn on a human body; a first light source unit which is provided on an inner surface of the main body and contacts the human body wearing the main body; a second light source unit which is provided on the inner surface of the main body and irradiates light in the same direction as the first light source unit; a light receiving unit which receives light from the first light source unit and the second light source unit; and a calculation unit which calculates a glucose level in blood based on the amount of light received by the light receiving unit and measures blood glucose using the calculated glucose level in the blood.

The first light source unit may irradiate light having a wavelength of 1850 to 1920 nm, and the second light source unit may irradiate light having a wavelength of 2050 to 2130 nm.

The main body may have a groove or a through hole, into which a finger or a wrist can be inserted, on a side thereof.

The calculation unit may calculate the glucose level in the blood using BloodGlucose = (Xₜ₁ x P1) + (Yₜ₂ x P2) + P3 by combining the amount of light X of the first light source unit received at a time a predetermined period of time t1 before a current time and the amount of light Y of the second light source unit received at a time a predetermined period of time t2 before the current time with unique constants P1, P2 and P3.

The light receiving unit may include: a light receiving sensor which receives light irradiated from the first light source unit or the second light source unit; a timing circuit which classifies an optical signal received by the light receiving sensor according to wavelength; a current-voltage converter which converts a current signal output from the light receiving sensor into a voltage signal; a first operational amplifier which amplifies a signal output from the current-voltage converter; a high-pass filter which passes a signal of 0.1 Hz or higher from among signals that passed through the first operational amplifier; a low-pass filter which passes a signal of 20 Hz or lower from among the signals that passed through the first operational amplifier; and a second operational amplifier which amplifies a signal that passed through the high-pass filter and the low-pass filter.

The apparatus may further include a body information detection unit which is provided on the inner surface of the main body to contact the human body and measures any one of electrocardiogram (ECG) and photoplethysmography (PPG), and the calculation unit may correct the calculated glucose level in the blood using a value detected by the body information detection unit.

The apparatus may further include a body information detection unit which is provided on the inner surface of the main body to contact the human body and measures any one of saturation of peripheral oxygen (SpO₂) and PPG, and the calculation unit may correct the calculated glucose level in the blood using a value detected by the body information detection unit.

The apparatus may further include a body information detection unit which is provided on the inner surface of the main body to contact the human body and measures any one of galvanic skin reflex (GSR) and body temperature, and the calculation unit may correct the calculated glucose level in the blood using a value detected by the body information detection unit.

The apparatus may further include a breathing pattern detection unit which is provided on an outer surface of the main body and measures a breathing pattern when placed adjacent to a respiratory system, and the calculation unit corrects the calculated glucose level in the blood using a value detected by the breathing pattern detection unit.

### [Advantageous Effects]

A blood glucose measurement method and apparatus using multiple body signals according to the present invention provide at least one of the following advantages.
First, since the same amount of light is absorbed by water from among light of a wavelength irradiated from a first light source unit and light of a wavelength irradiated from a second light source unit, a blood glucose level can be accurately measured by excluding the amount of light absorbed by water.
Second, since the same amount of light is lost by water from among light irradiated from the first light source unit and light irradiated from the second light source, a difference in the amount of light received by a light receiving unit is purely attributable to a difference in blood glucose level. Therefore, a blood glucose level is hardly affected by measurement location or body temperature.
Third, since the blood glucose measurement apparatus is wearable on a human body such as a wrist or a finger, a blood glucose level of a subject wearing the blood glucose measurement apparatus can be continuously monitored. Therefore, a sudden change in blood glucose level can be dealt with.
Fourth, a glucose level at the time of measurement is not used to calculate a glucose level. Instead, the glucose level is calculated based on a glucose value before a blood glucose level is changed by the external environment. Therefore, more accurate blood glucose measurement is possible.
Fifth, a blood glucose level measured by calculating a glucose level is corrected using a body signal such as electrocardiogram (ECG) or photoplethysmography (PPG). Therefore, a more accurate blood glucose level can be identified.
Sixth, a blood glucose level measured by calculating a glucose level is corrected using a body signal such as saturation of peripheral oxygen (SpO₂) or PPG. Therefore, a more accurate blood glucose level can be identified.
Seventh, a blood glucose level measured by calculating a glucose level is corrected using a body signal such as galvanic skin reflex (GSR) or body temperature. Therefore, a more accurate blood glucose level can be identified.
Eighth, a blood glucose level measured by calculating a glucose level is corrected using a body signal such as a breathing pattern. Therefore, a more accurate blood glucose level can be identified.

However, the effects of the present invention are not restricted to the one set forth herein. The above and other effects of the present invention will become more apparent to one of daily skill in the art to which the present invention pertains by referencing the claims.

### [Description of Drawings]

The above and other aspects and features of the present invention will become more apparent by describing in detail exemplary embodiments thereof with reference to the attached drawings, in which:
FIG. 1 is a graph illustrating optical wavelength versus absorbance to explain a blood glucose measurement principle of the present invention;
FIG. 2 is a graph illustrating optical wavelengths detected in response to the irradiation of light having a wavelength of 1880 nm and light having a wavelength of 2080 nm.;
FIG. 3 is a graph illustrating a subject's blood glucose level versus absorbance in response to light having a wavelength of 1880 nm;
FIG. 4 is a graph illustrating a subject's blood glucose level versus absorbance in response to light having a wavelength of 2080 nm;
FIG. 5 is a cross-sectional view of a blood glucose measurement method and apparatus using multiple body signals according to an embodiment of the present invention;
FIG. 6 illustrates the configuration of the blood glucose measurement method and apparatus of FIG. 5.
FIG. 7 illustrates the detailed configuration of a light receiving unit according to an embodiment of the present invention; and
FIG. 8 is a flowchart illustrating a process of measuring blood glucose according to an embodiment of the present invention.

### <Explanation of Reference numerals designating the Major elements of the Drawings>

100: main body 111: first light source unit
113: second light source unit 120: light receiving unit
121: light receiving sensor 122: timing circuit
123: current-voltage converter 124: first operational amplifier
125: high-pass filter 126: low-pass filter
127: second operational amplifier 130: calculation unit
141: body information detection unit 143: breathing pattern detection unit
150: groove

### [Mode for Invention]

Hereinafter, exemplary embodiments of the present invention will be described in further detail with reference to the attached drawings.

A blood glucose measurement principle of the present invention is to measure a blood glucose level based on the amount of light absorbed in a human body from among light irradiated to the human body. More specifically, water exists not only in blood but also in bones and cortical layers of the human body. Therefore, water is inappropriate to be used as a parameter. In the present invention, a blood glucose level is detected not by measuring mixed light of two or more water-containing materials with different absorbances. Instead, since the concentration of glucose in blood varies according to the blood glucose level, the absorbance of glucose only is used to detect glucose content in blood.

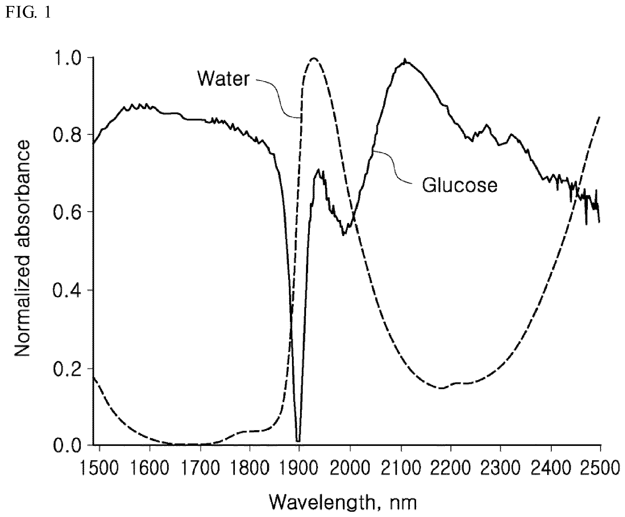

FIG. 1 is a graph illustrating optical wavelength versus absorbance to explain a blood glucose measurement principle of the present invention.

Referring to FIG. 1, water has the same absorbance of approximately 30 % for light having a wavelength of approximately 1880 nm and light having a wavelength of approximately 2080 nm. On the other hand, glucose has different absorbance values for the light having the above wavelengths. While glucose, like water, absorbs approximately 30 % of the light having the wavelength of 1880 nm, it absorbs approximately 90 % of the light having the wavelength of 2080 nm. Therefore, if the light having the wavelength of 1880 nm and the light having the wavelength of 2080 nm are alternately irradiated to the same location on the human body, they may transmit through the human body to be received in different amounts. It can be concluded that this difference in the amount of light received results from a difference in the absorbance of glucose.

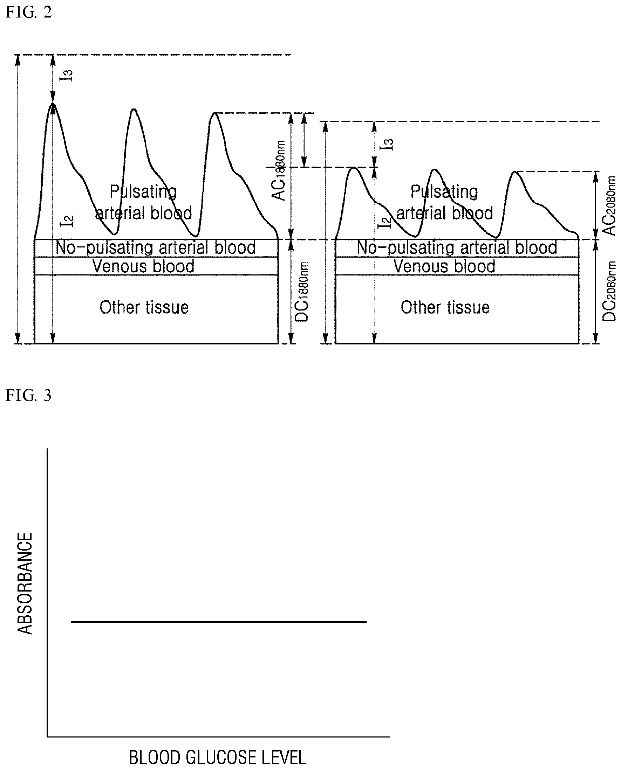

FIG. 2 is a graph illustrating optical wavelengths detected in response to the irradiation of light having a wavelength of 1880 nm and light having a wavelength of 2080 nm.

After each of the light having the wavelength of 1880 nm and the light having the wavelength of 2080 nm is irradiated to the same location on the human body, if light that passed through the human body is received, the results as shown in FIG. 2 can be obtained. The received optical signal of each wavelength is divided into an alternating current (AC) component and a direct current (DC) component.

Here, the AC component indicates the amount of light absorbed by blood passing through peripheral blood vessels, and the DC component indicates the amount of light absorbed by capillaries or tissue such as muscles, epidermis and bones.

Since water has the same absorbance of approximately 30 % for both the light having the wavelength of 1880 nm and the light having the wavelength of 2080 nm, the amount of light lost by water from among the amount of light lost as light irradiated from a light source unit passes through the human body may be the same for both the light having the wavelength of 1880 nm and the light having the wavelength of 2080 nm. Therefore, the DC components of the received signals may be presented as illustrated in the drawing based on an analysis of the received signals.

On the other hand, glucose has far higher absorbance than water for the light having the wavelength of 2080 nm. Therefore, as a blood glucose level increases, the amplitude of the AC component detected is smaller than that for the light having the wavelength of 1880 nm.

Based on the amounts of light detected for the above two wavelengths, a model for estimating a blood glucose level can be established as follows.

Blood Glucose =(Xₜ₁ x P1) + (Yₜ₂ x P2) + P3, ... (1)

where X is the amount of light received from among the light having the wavelength of 1880 nm, and Y is the amount of light received from among the light having the wavelength of 2080 nm.

In addition, t1 and t2 are predetermined periods of time before a current time when a blood glucose level is measured. The amounts of light X and Y measured the predetermined periods of time t1 and t2 before the current time are used to measure a blood glucose level because it takes approximately 20 to 30 seconds for blood released from the heart to return to the heart and because it takes time for a stimulus, such as meal intake, exercise or insulin secretion, to affect glucose in blood. Therefore, to detect a blood glucose level before the blood glucose level is changed by the above stimulus, the amounts of light X and Y measured the periods of time t1 and t2 before the current time are used. The periods of time t1 and t2 may be variables that can be set directly by a subject or automatically by a system that executes blood glucose measurement according to the situation and environment in which blood glucose measurement is needed.

Further, P1, P2 and P3 are constants used to calculate a glucose level based on the amounts of light received and may be defined through statistical analysis or mathematical modeling.

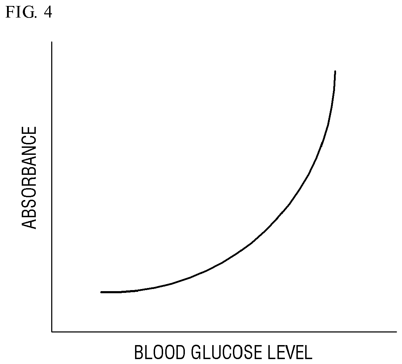

FIG. 3 is a graph illustrating a subject's blood glucose level versus absorbance in response to light having a wavelength of 1880 nm. FIG. 4 is a graph illustrating a subject's blood glucose level versus absorbance in response to light having a wavelength of 2080 nm.

Referring to FIG. 3, the absorbance of glucose for the light having the wavelength of 1880 nm remains constant regardless of whether a blood glucose level is high or low.

Referring to FIG. 4, the absorbance of glucose for the light having the wavelength of 2080 nm increases in proportion to an increase in blood glucose level.

The proportion of glucose in the blood of a subject with a high blood glucose level is higher than a normal level. Therefore, while the amount of light received after the light having the wavelength of 1880 nm is irradiated to the body of the subject with a high blood glucose level is not much different from the amount of light received after the light having the wavelength of 1880 nm is irradiated to the body of a subject with a normal blood glucose level, the amount of light received after the light having the wavelength of 2080 nm is irradiated to the body of the subject with a high blood glucose level is smaller than the amount of light received after the light having the wavelength of 2080 nm is irradiated to the body of the subject with a normal blood glucose level.

In this regard, the amount of light received after the irradiation of the light having the wavelength of 1880nm can be used as a basis for identifying the absorbance of water in the body of a subject whose blood glucose level is measured. In addition, the amount of light received after the irradiation of the light having the wavelength of 2080 nm can be used as a value for detecting whether a blood glucose level is higher or lower than that calculated based on the amount of light received after the irradiation of the light having the wavelength of 1880 nm.

A blood glucose measurement method and apparatus using multiple body signals and based on the above blood glucose measurement theory according to an embodiment of the present invention will hereinafter be described.

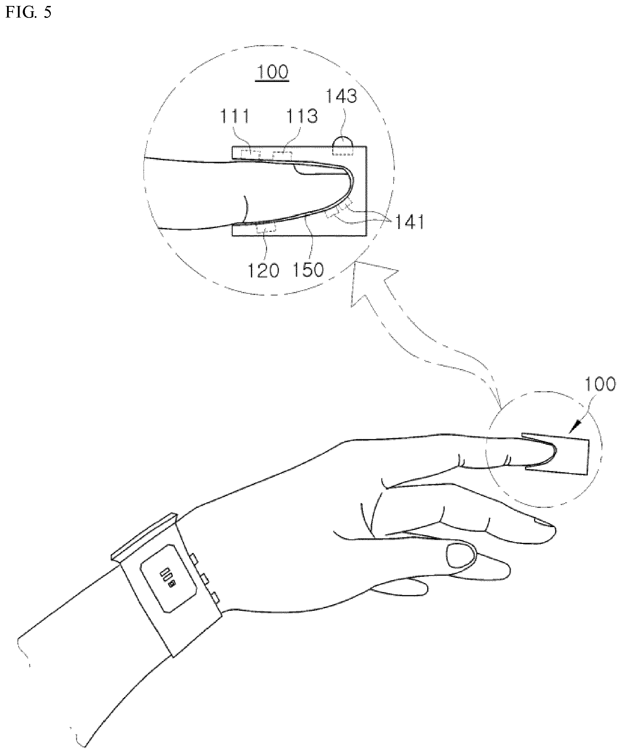

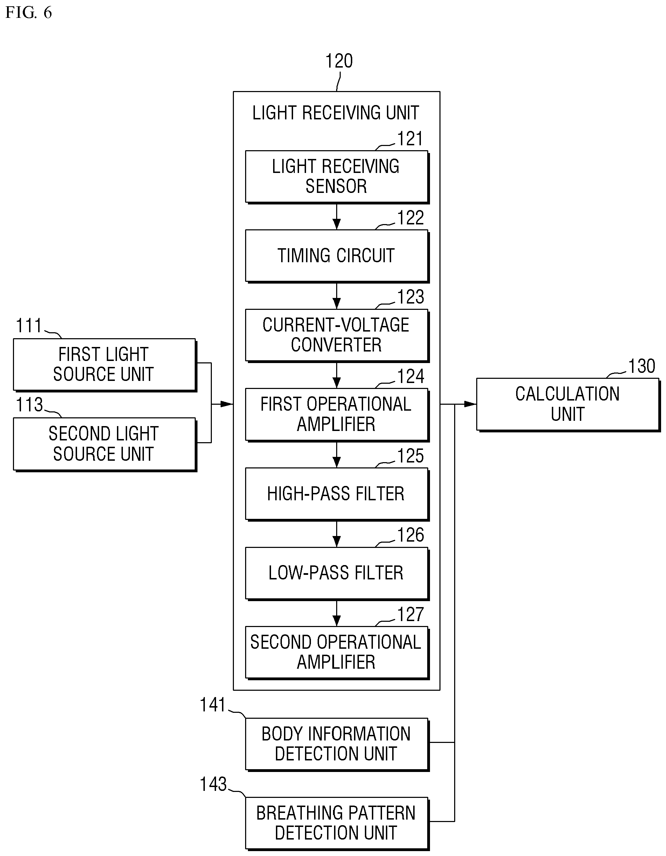

FIG. 5 is a cross-sectional view of a blood glucose measurement method and apparatus using multiple body signals according to an embodiment of the present invention. FIG. 6 illustrates the configuration of the blood glucose measurement method and apparatus of FIG. 5.

Referring to FIGS. 5 and 6, the blood glucose measurement apparatus using multiple body signals according to the current embodiment includes a main body 100 which can be worn on a human body, a first light source unit 111 which is provided on an inner surface of the main body 100 and contacts the human body wearing the main body 100, a second light source unit 113 which is provided on the inner surface of the main body 100 and irradiates light in the same direction as the first light source unit 111, a light receiving unit 120 which receives light from the first light source unit 111 and the second light source unit 113, and a calculation unit 130 which calculates a glucose level in blood based on the amount of light received by the light receiving unit 120 and measures blood glucose using the calculated glucose level.

The main body 100 may have a shape that allows it to be worn on a human body. For example, the main body 100 may be shaped like a bracelet having a through hole so as to be worn on the wrist or, as in the current embodiment, shaped like a thimble having a groove 150 into which a finger can be inserted.

In addition, to receive light irradiated from the first light source unit 111 and the second light source unit 113, the light receiving unit 120 may be provided inside the main body 100 at a location facing the first light source unit 111 and the second light source unit 113.

In particular, the first light source unit 111 may irradiate light having a wavelength of 1850 to 1920 nm, and the second light source unit 113 may irradiate light having a wavelength of 2050 to 2130 nm. The light having the wavelength of 1850 to 2130 nm is light in an infrared wavelength region and cannot be perceived by the eyes.

The first light source unit 111 has the wavelength of 1850 to 1920 nm because glucose and water have similar absorbance values in the above wavelength region. Therefore, the first light source unit 111 can be used to measure the absorbance of water in the body of a subject and identify body characteristics of the subject so as to measure blood glucose.

In addition, the second light source unit 113 has the wavelength of 2050 to 2130 nm because the absorbance of water in the above wavelength region is similar to that in the wavelength region of the first light source unit 111. Therefore, the absorbance of water can be identified using the second light source unit 113. On the other hand, since the absorbance of glucose is higher in the wavelength region of the second light source unit 113 than in the wavelength region of the first light source unit 111, a glucose level can be inferred using this difference.

When light irradiated from the first light source unit 111 and the second light source unit 113 is received by the light receiving unit 120, the calculation unit 130 may calculate a blood glucose level using Equation (1) by combining the amount of light X of the first light source unit 111 received at a time a predetermined period of time t1 before a current time and the amount of light Y of the second light source unit 113 received at a time a predetermined period of time t2 before the current time with unique constants P1, P2 and P3 used to measure a glucose level in blood.

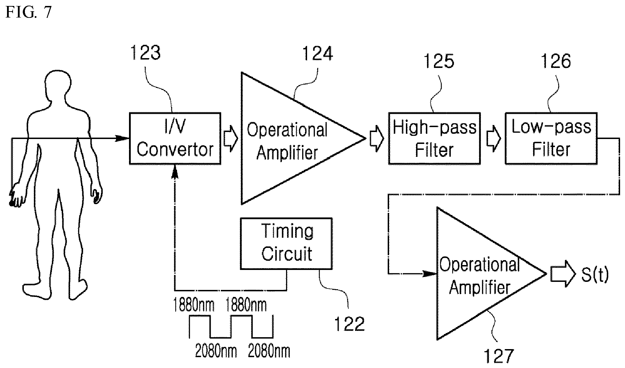

FIG. 7 illustrates the detailed configuration of a light receiving unit according to an embodiment of the present invention.

Referring to FIG. 7, the light receiving unit 120 may include a light receiving sensor 121 which receives light irradiated from the first light source unit 111 or the second light source unit 113, a timing circuit 122 which classifies an optical signal received by the light receiving sensor 121 according to wavelength, a current (I)-voltage (V) converter 123 which converts a current signal output from the light receiving sensor 121 into a voltage signal, a first operational amplifier 124 which amplifies a signal output from the I-V converter 123, a high-pass filter 125 which passes a signal of 0.1 Hz or higher from among signals that passed through the first operational amplifier 124, a low-pass filter 126 which passes a signal of 20 Hz or lower from among the signals that passed through the first operational amplifier 124, and a second operational amplifier 127 which amplifies a signal that passed through the high-pass filter 125 and the low-pass filter 126.

A signal that passed through the timing circuit 122 is amplified by the first operational amplifier 124 such that a voltage gain of the signal becomes ten times, and the amplified signal is passed through the high-pass filter 125 which passes a signal of 0.1 Hz or higher and the low-pass filter 126 which passes a signal of 20 Hz or lower.

Then, the signal is amplified again by the second operational amplifier 127 such that the voltage gain of the signal becomes 50 times. Ultimately, the voltage gain of the signal that passed through the timing circuit 122 is amplified 500 times to 25 dB.

In addition, to minimize an error in a glucose level calculated by the calculation unit 130, the calculated glucose level may be corrected using a blood glucose level obtained by detecting different types of body information. To this end, a body information detection unit 141 which measures any one of electrocardiogram (ECG) and photoplethysmography (PPG) may further be provided on the inner surface of the main body 100 to contact the human body, and the calculation unit 130 may correct the calculated glucose level using a value detected by the body information detection unit 141.

An ECG is a waveform that represents the vector sum of action potentials generated by a special excitatory & conductive system. That is, the ECG is a vector sum signal, measured using electrodes attached onto the human body, of action potentials generated by each component of the heart such as sinoatrial (SA) node, atrioventricular (AV) node, His bundle, bundle branches, Purkinje fibers, etc.

Generally, an ECG signal is obtained using a standard limb lead method that utilizes four electrodes.

A PPG is a pulse wave signal measured in peripheral blood vessels when blood ejected during ventricular systole is delivered to the peripheral blood vessels. To measure a PPG signal, optical characteristics of biological tissue are used.

Generally, pulse transit time (PTT) or pulse wave velocity (PWV) is extracted by analyzing the correlation between a PPG signal and an ECG signal, instead of the PPG signal only, and cardiovascular diseases are diagnosed based on the extracted PTT or PWV. To this end, a characteristic point is obtained by performing a quadratic differential on the PPG signal, and then a time interval from a peak (R wave) of the ECG signal is measured.

A reduction of glucose in blood increases a heart rate in order to supply more blood, and an ECG changes according to a change in blood glucose. For example, a reduction in an R-R interval value in an ECG waveform may increase the heart rate. Therefore, the reduction in the R-R interval value can be understood as a fall in blood glucose level. In addition, a high frequency (HF)/low frequency (LF) ratio, which represents a change in ECG, and a change in the time range of a QRST waveform may also vary according to a change in blood glucose level. Therefore, these body signals and the R-R interval can be used as constants that are proportional to a blood glucose level in order to increase the accuracy of measured blood glucose levels.

In addition, the body information detection unit 141 which measures any one of saturation of peripheral oxygen (SpO₂) and PPG may further be provided on the inner surface of the main body 100 to contact the human body, and the calculation unit 130 may correct the calculated glucose level using a value detected by the body information detection unit 141.

SpO₂ is a body signal indicating oxygen content in hemoglobin from among various components of blood.

A lack of insulin in the human body increases the heart rate, thus lowering a SpO₂ value and changing a breathing cycle.

In addition, a body information detection unit 141 which measures any one of galvanic skin reflex (GSR) and body temperature may further be provided on the inner surface of the main body 100 to contact the human body, and the calculation unit 130 may correct the calculated glucose level using a value detected by the body information detection unit 141.

GSR is a body signal measurement method used to measure the psychological and physical arousal state of a subject based on the electrical conductivity of the skin because sweat glands of the skin are under the control of a sympathetic nervous system.

A drop in blood glucose increases the heart rate, causing cold sweating. Cold sweating is a phenomenon that occurs when contraction and dilatation of the heart are caused not by an external stimulus but by a hormone imbalance due to a lack of insulin. Such a change in the human body causes a change in GSR. Therefore, if the change in GSR is measured, a value proportional to a blood glucose level can be obtained.

Body temperature falls as blood glucose falls. Therefore, if a fall in body temperature being monitored is detected, it can be understood as a fall in blood glucose. That is, body temperature can be used to measure blood glucose.

In addition, a breathing pattern detection unit 143 which measures a breathing pattern when placed adjacent to a respiratory system may further be provided on an outer surface of the main body 100, and the calculation unit 130 may correct the calculated glucose level using a value detected by the breathing pattern detection unit 143.

A lack of insulin in the human body increases the heart rate, thus lowering the SpO₂ value and increasing the breathing cycle. Therefore, if the main body 100 is placed adjacent to the respiratory system during blood glucose measurement, a breathing pattern of a subject can be measured by the breathing pattern detection unit 143 provided on the outer surface of the main body 100. The detected breathing pattern can be used as an auxiliary means of inferring blood glucose.

Hereinafter, a method of measuring blood glucose by utilizing a blood glucose measurement apparatus using multiple body signals according to an embodiment of the present invention will be described.

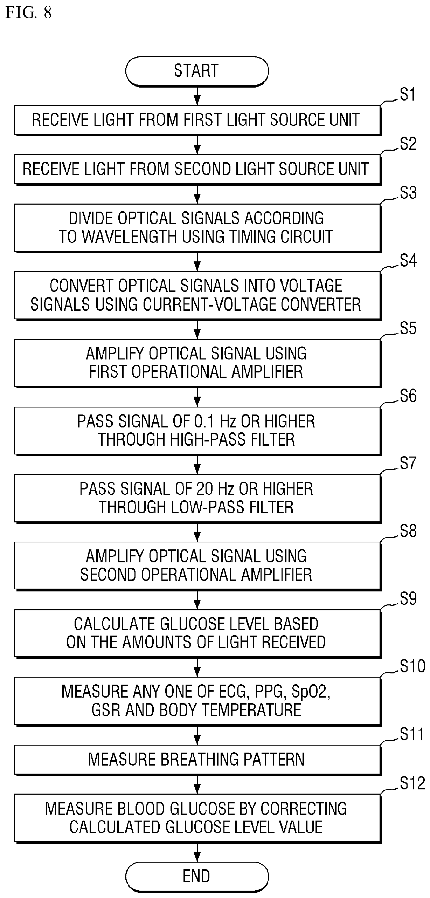

FIG. 8 is a flowchart illustrating a process of measuring blood glucose according to an embodiment of the present invention.

The present invention includes receiving first light having a wavelength of 1850 to 1920 nm from a first light source unit 111 by using a light receiving unit 120 (operation S01), receiving second light having a wavelength of 2050 to 2130 nm from a second light source unit 113 by using the light receiving unit 120 (operation S02), and calculating a glucose level in blood based on the amounts of the first light and the second light received by the light receiving unit 120 and measuring blood glucose based on the calculated glucose level by using a calculation unit 130 (operation S09).

In addition, the present invention may further include, after the receiving of the first light and the second light using the light receiving unit 120 (operations S01 and S02), dividing the received optical signals into the first light and the second light using a timing circuit 122 (operation S03), converting the optical signals divided into the first light and the second light by the timing circuit 122 into voltage signals using an I-V converter 123 (operation S04), amplifying a signal output from the I-V converter 123 using a first operational amplifier 124 (operation S05), passing a signal of 0.1 Hz or higher through a high-pass filter 125 from among signals that passed through the first operational amplifier 124 (operation S06), passing a signal of 20 Hz or lower through a low-pass filter 126 from among the signals that passed through the first operational amplifier 124 (operation S07), and amplifying a signal that passed through the high-pass filter 125 and the low-pass filter 126 using a second operational amplifier 127 (operation S08).

In addition, the calculation unit 130 may calculate the glucose level after excluding 20 to 40 % of light absorbed and thus lost by water in a human body from the amount of light received by the light receiving unit 120.

In addition, the calculation unit 130 may calculate the glucose level in the blood as in Equation (1) by combining the amount of light X of the first light source unit 111 received at a time a predetermined period of time t1 before a current time and the amount of light Y of the second light source unit 113 received at a time a predetermined period of time t2 before the current time with unique constants P1, P2 and P3 used to measure the glucose level in the blood.

In addition, the present invention may further include, after the calculating of the glucose level in the blood using the calculation unit 130, measuring any one of ECG and PPG using a body information detection unit 141 (operation S10) and correcting the glucose level in the blood calculated by the calculation unit 130 with a value detected by the body information detection unit 141 (operation S12).

In addition, the present invention may further include, after the calculating of the glucose level in the blood using the calculation unit 130, measuring any one of SpO₂ and PPG using a body information detection unit 141 (operation S10) and correcting the glucose level in the blood calculated by the calculation unit 130 using a value detected by the body information detection unit 141 (operation S12).

In addition, the present invention may further include, after the calculating of the glucose level in the blood using the calculation unit 130, measuring any one of GSR and body temperature using a body information detection unit 141 (operation S10) and correcting the glucose level in the blood calculated by the calculation unit 130 using a value detected by the body information detection unit 141 (operation S12).

In addition, the present invention may further include, after the calculating of the glucose level in the blood using the calculation unit 130, measuring a breathing pattern using a breathing pattern detection unit 143 (operation S11) and correcting the glucose level in the blood calculated by the calculation unit 130 using a value detected by the breathing pattern detection unit 143 (operation S12).

Although the exemplary embodiments of the present invention have been described, the present invention may employ various modifications, changes and equivalents. It will be apparent that the present invention may be equally applied by appropriately modifying the embodiments. Accordingly, the descriptions are not intended to limit the scope of the present invention defined by the appended claims.

## Claims

1. A blood glucose measurement apparatus using multiple body signals, the apparatus comprising:
a main body which can be worn on a human body;
a first light source unit which is provided on an inner surface of the main body and contacts the human body wearing the main body;
a second light source unit which is provided on the inner surface of the main body and irradiates light in the same direction as the first light source unit;
a light receiving unit which receives light from the first light source unit and the second light source unit; and
a calculation unit which calculates a glucose level in blood based on the amount of light received by the light receiving unit and measures blood glucose using the calculated glucose level in the blood,
wherein the calculation unit calculates the glucose level in the blood using BloodGlucose = (Xₜ₁ x P1) + (Yₜ₂ x P2) + P3 by combining the amount of light X of the first light source unit received at a time a predetermined period of time t1 before a current time and the amount of light Y of the second light source unit received at a time a predetermined period of time t2 before the current time with unique constants P1, P2 and P3.

2. The apparatus of claim 1, wherein the first light source unit irradiates light having a wavelength of 1850 to 1920 nm, and the second light source unit irradiates light having a wavelength of 2050 to 2130 nm.

3. The apparatus of claim 1, wherein the main body has a groove or a through hole, into which a finger or a wrist can be inserted, on a side thereof.

4. The apparatus of claim 1, wherein the light receiving unit comprises:
a light receiving sensor which receives light irradiated from the first light source unit or the second light source unit;
a timing circuit which classifies an optical signal received by the light receiving sensor according to wavelength;
a current-voltage converter which converts a current signal output from the light receiving sensor into a voltage signal;
a first operational amplifier which amplifies a signal output from the current-voltage converter;
a high-pass filter which passes a signal of 0.1 Hz or higher from among signals that passed through the first operational amplifier;
a low-pass filter which passes a signal of 20 Hz or lower from among the signals that passed through the first operational amplifier; and
a second operational amplifier which amplifies a signal that passed through the high-pass filter and the low-pass filter.

5. The apparatus of claim 1, further comprising a body information detection unit which is provided on the inner surface of the main body to contact the human body and measures any one of electrocardiogram (ECG) and photoplethysmography (PPG), and
the calculation unit corrects the calculated glucose level in the blood using a value detected by the body information detection unit.

6. The apparatus of claim 1, further comprising a body information detection unit which is provided on the inner surface of the main body to contact the human body and measures any one of saturation of peripheral oxygen (SpO₂) and PPG, and the calculation unit corrects the calculated glucose level in the blood using a value detected by the body information detection unit.

7. The apparatus of claim 1, further comprising a body information detection unit which is provided on the inner surface of the main body to contact the human body and measures any one of galvanic skin reflex (GSR) and body temperature, and
the calculation unit corrects the calculated glucose level in the blood using a value detected by the body information detection unit.

8. The apparatus of claim 1, further comprising a breathing pattern detection unit which is provided on an outer surface of the main body and measures a breathing pattern when placed adjacent to a respiratory system, and
the calculation unit corrects the calculated glucose level in the blood using a value detected by the breathing pattern detection unit.

9. A blood glucose measurement apparatus using multiple body signals, the apparatus comprising:
a main body having a groove or a through hole into which a finger or a wrist can be inserted;
a first light source unit which is provided on an inner surface of the main body to contact a human body wearing the main body and irradiates light having a wavelength of 1850 to 1920 nm;
a second light source unit which is provided on the inner surface of the main body and irradiates light having a wavelength of 2050 to 2130 nm in the same direction as the first light source unit;
a light receiving unit which receives light from the first light source unit and the second light source unit;
a body information detection unit which is provided on the inner surface of the main body to contact the human body and measures any one of ECG, PPG, SpO₂, GSR, and body temperature;
a breathing pattern detection unit which is provided on an outer surface of the main body and measures a breathing pattern when placed adjacent to a respiratory system; and
a calculation unit which measures blood glucose by calculating a glucose level in blood using BloodGlucose = (Xₜ₁ x P1) + (Yₜ₂ x P2) + P3 by combining the amount of light X of the first light source unit received at a time a predetermined period of time t1 before a current time and the amount of light Y of the second light source unit received at a time a predetermined period of time t2 before the current time with unique constants P1, P2 and P3,
wherein the calculation unit corrects the calculated glucose level in the blood using values detected by the body information detection unit and the breathing pattern detection unit, and the light receiving unit comprises a light receiving sensor which receives light irradiated from the first light source unit or the second light source unit, a timing circuit which classifies an optical signal received by the light receiving sensor according to wavelength, a current-voltage converter which converts a current signal output from the light receiving sensor into a voltage signal, a first operational amplifier which amplifies a signal output from the current-voltage converter, a high-pass filter which passes a signal of 0.1 Hz or higher from among signals that passed through the first operational amplifier, a low-pass filter which passes a signal of 20 Hz or lower from among the signals that passed through the first operational amplifier, and a second operational amplifier which amplifies a signal that passed through the high-pass filter and the low-pass filter.

10. A blood glucose measurement method using multiple body signals, the method comprising:
receiving first light having a wavelength of 1850 to 1920 nm from a first light source unit by using a light receiving unit;
receiving second light having a wavelength of 2050 to 2130 nm from a second light source unit by using the light receiving unit; and
calculating a glucose level in blood based on the amounts of the first light and the second light received by the light receiving unit and measuring blood glucose based on the calculated glucose level by using a calculation unit,
wherein the calculation unit calculates the glucose level in the blood after excluding 20 to 40 % of light absorbed and thus lost by water in the human body from the amount of light received by the light receiving unit.

11. The method of claim 10, comprising, after the receiving of the first light and the second light using the light receiving unit:
dividing the received optical signals into the first light and the second light using a timing circuit;
converting the optical signals divided into the first light and the second light by the timing circuit into voltage signals using a current-voltage converter;
amplifying a signal output from the current-voltage converter using a first operational amplifier;
passing a signal of 0.1 Hz or higher through a high-pass filter from among signals that passed through the first operational amplifier;
passing a signal of 20 Hz or lower through a low-pass filter from among the signals that passed through the first operational amplifier; and
amplifying a signal that passed through the high-pass filter and the low-pass filter using a second operational amplifier.

12. The method of claim 10, wherein the calculation unit calculates the glucose level in the blood using BloodGlucose = (Xₜ₁ x P1) + (Yₜ₂ x P2) + P3 by combining the amount of light X of the first light source unit received at a time a predetermined period of time t1 before a current time and the amount of light Y of the second light source unit received at a time a predetermined period of time t2 before the current time with unique constants P1, P2 and P3.

13. The method of claim 10, further comprising, after the calculating of the glucose level in the blood using the calculation unit:
measuring any one of ECG and PPG using a body information detection unit; and
correcting the glucose level in the blood calculated by the calculation unit using a value detected by the body information detection unit.

14. The method of claim 10, further comprising, after the calculating of the glucose level in the blood using the calculation unit:
measuring any one of SpO₂ and PPG using a body information detection unit; and
correcting the glucose level in the blood calculated by the calculation unit using a value detected by the body information detection unit.

15. The method of claim 10, further comprising, after the calculating of the glucose level in the blood using the calculation unit:
measuring any one of GSR and body temperature using a body information detection unit; and
correcting the glucose level in the blood calculated by the calculation unit using a value detected by the body information detection unit.

16. The method of claim 10, further comprising, after the calculating of the glucose level in the blood using the calculation unit:
measuring a breathing pattern using a breathing pattern detection unit;
and
correcting the glucose level in the blood calculated by the calculation unit using a value detected by the breathing pattern detection unit.

17. A blood glucose measurement method using multiple body signals, the method comprising:
receiving first light having a wavelength of 1850 to 1920 nm from a first light source unit by using a light receiving unit;
receiving second light having a wavelength of 2050 to 2130 nm from a second light source unit by using the light receiving unit;
dividing signals of the first light and the second light received by the light receiving unit into the first light and the second light using a timing circuit;
converting the optical signals divided into the first light and the second light by the timing circuit into voltage signals using a current-voltage converter;
amplifying a signal output from the current-voltage converter using a first operational amplifier;
passing a signal of 0.1 Hz or higher through a high-pass filter from among signals that passed through the first operational amplifier;
passing a signal of 20 Hz or lower through a low-pass filter from among the signals that passed through the first operational amplifier;
amplifying a signal that passed through the high-pass filter and the low-pass filter using a second operational amplifier;
subtracting 20 to 40 % of light absorbed and thus lost by water in the human body from the amount of light received by the light receiving unit;
calculating a glucose level in blood using BloodGlucose = (Xₜ₁ x P1) + (Yt₂ x P2) + P3 by combining the amount of light X of the first light source unit received at a time a predetermined period of time t1 before a current time and the amount of light Y of the second light source unit received at a time a predetermined period of time t2 before the current time with unique constants P1, P2 and P3 using a calculation unit;
measuring any one of ECG, PPG, SpO₂, GSR, and body temperature using a body information detection unit;
measuring a breathing pattern using a breathing pattern detection unit;
and
correcting the glucose level in the blood calculated by the calculation unit using values detected by the body information detection unit and the breathing pattern detection unit.
